(19) Europäisches Patentamt
European Patent Office
Office européen des brevets

(11) Publication number: 0 203 184
B1

(12) # EUROPEAN PATENT SPECIFICATION

(45) Date of publication of the patent specification: **27.09.89**

(51) Int. Cl.⁴: **C 08 G 85/00**, C 08 G 77/32, A 61 L 27/00, A 61 F 2/52, C 08 J 3/00

(21) Application number: **86900391.3**

(22) Date of filing: **25.11.85**

(86) International application number: **PCT/US 85/02342**

(87) International publication number: **WO 86/03503 (19.06.86 Gazette 86/13)**

(54) **METHOD OF MANUFACTURING ARTICLES CONTAINING STABILIZED POLYMERS (GELS).**

(30) Priority: **03.12.84 US 677681**

(43) Date of publication of application: **03.12.86 Bulletin 86/49**

(45) Publication of the grant of the patent: **27.09.89 Bulletin 89/39**

(84) Designated Contracting States: **DE FR GB IT NL SE**

(56) References cited: **none**

(73) Proprietor: **AMERICAN TELEPHONE AND TELEGRAPH COMPANY, 550 Madison Avenue, New York, NY 10022 (US)**

(72) Inventor: **WONG, Ching-Ping, 11 Wexford Drive, Lawrenceville, NJ 08548 (US)**

(74) Representative: **Johnston, Kenneth Graham et al, AT&T (UK) LTD. AT&T Intellectual Property Division 5 Mornington Road, Woodford Green Essex, IG8 OTU (GB)**

EP 0 203 184 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

This invention relates to a method manufacturing articles containing stabilized resin formulations formed by curing of prepolymers in the presence of a catalyst and articles made therefrom.

### Background of the Invention

Silicone polymers are well known in the polymer art. They enjoy a wide variety of uses such as encapsulants and coatings for the electronics industry, sealants and greases, medical implants and in the media for certain types of touch sensitive displays. In many instances, the silicone polymer is formed by polymerization of a silicone or mixture of silicones in the presence of a catalyst. Further, in many instances it is desired to stop the polymerization process in order to achieve a silicone polymer which has a certain desired consistency such as a gel consistency. This is true, for example, for both silicone implants used in the medical industry for such things as breast implants, as well as the silicone formulation employed in touch sensitive screen for electro-optical display devices. A problem that has been found to exist with such silicone is that with time, the curing process continues resulting in an undesirable hardening or thickening so as to change the consistency of the silicone from the desired consistency to one that is undesirable.

It was known from US-A-4 203 913 to stabilize organopolysiloxanes against undesired post-reactions by deactivating the phosphorus compound catalyst contained therein with ammonia or amines.

### Summary of the Invention

According to the present invention there is provided a method as claimed in claim 1.

This invention is based on a discovery of a means for substantially terminating the polymerization process after the desired consistency is reached so as to prevent the hardening that occurs with age in polymer formulations, such as silicone, cured by means of a catalyst. The invention includes the method of producing articles containing a polymer of a desired consistency. The method involves curing a polymer formulation in the presence of a catalyst so as to form a desired consistency and then treating the polymerized material so as to effectively deactivate the catalyst or cured resin without degrading the polymer. The resulting polymer of the desired consistency therefore contains a catalyst or cured resin which has been deactivated such that further polymerization is substantially reduced. Such polymerized material can then be employed in the manufacture of articles such as breast implants and touch sensitive screens as examples.

### Brief Description of the Drawings

Fig. 1 is a side elevational cross-sectional view of a touch sensitive screen; and

Figs. 2–4 show that plot of percent transmission vs. wavenumber obtained from the FT-IR spectrophotometric analysis of a newly produced touch sensitive, silicone gelfilled screen (Fig. 2); a screen after exposure to moisture (Fig. 3); and a screen after treatment with $NH_3$ followed by exposure to moisture (Fig. 4).

### Detailed Description

While this invention is described in terms of forming silicone gels and articles incorporating such gels from the polymerization of mixtures of silicone materials in the presence of a platinum catalyst, it should be understood that the concept herein, involving the deactivation of the catalyst or cured resin in order to insure the cessation of further polymerization or crosslinking subsequent to reaching the desired consistency of material, is equally applicable to any silicone formulation which is polymerized or crosslinked.

One method of forming silicone gels, for example, is the reaction of two silicone polymers having an addition reaction curing system in the presence of a platinum catalyst. For example, upon mixing a silicone hydride with a silicone polymer containing a vinyl group either at the end of a chain or along the siloxane chain (pendant groups), in the presence of a small amount of a platinum catalyst, e.g., in the order of about 1–10 ppm, an addition reaction occurs. When the vinyl groups are at the end of a chain, chain extension results while if the vinyl groups are along the chain a higher density crosslinking reaction results. Examples of such reactions are given below:

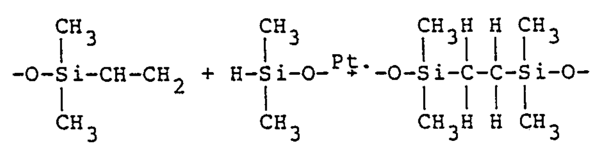

End group reaction (lengthen molecule)

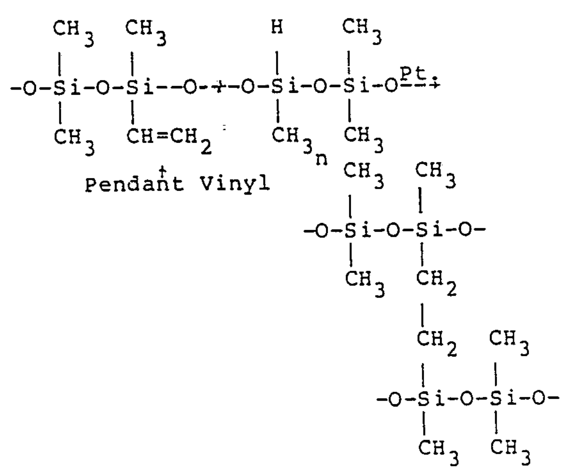

Higher Cross-link density reaction.

Another possible reaction mechanism involving the cured silicone gel which could lead to continued crosslinking is shown below:

(a)

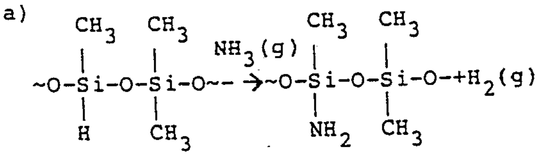


cured gel (Reactive) amonia siloxane (Still Reactive)

Theoretically, there are no volatile byproducts produced during this cure. However, hydrogen gases are always obtained during the cure due to other side reactions. If the number of Si–H groups of a silicone hydride exceeds the number of vinyl groups in the vinyl containing silicone compound such that the ratio of Si–H/Si–vinyl is greater than one, a gel results. If on the other hand, the ratio is less than one, i.e., the number of Si–vinyl groups exceeds the number of Si-H groups, then an elastomer is produced.

Application for these versatile materials are numerous and span various industries including the automative, aerospace, applicance, electrical and medical industries. Generally, lightly crosslinked addition reaction systems can be engineered to produce soft, clear vibration absorbing gels which may either be used in the electronics industry to pot delicate assemblies or as the primary component of a touch screen cathode ray tube which operates based upon the deflection of light upon pressure placed on the screen. Another use of such gel is in the implantable breast prosthesis in the medical industry. Increased crosslinking leads to harder and tougher silicones which enjoy many other uses as is well known in the art.

A problem which exists in articles made from silicone gels is that after time, there is often a tendency for the gel to undesirably harden and also to produce gas bubbles. The present inventor has discovered that this hardening is due primarily to the diffusion of water vapor into the gel and the reaction of this water vapor in the presence of the active catalyst, e.g., platinum, to form active siloxy or silanol groups which react either with each other or with unreacted hydrogen atoms contained on silicone hydride bonds so as to induce further crosslinking. These reactions result in the formation of hydrogen gas which is the cause of the bubbles. Typical reactions of the type contemplated is shown below:

(1)

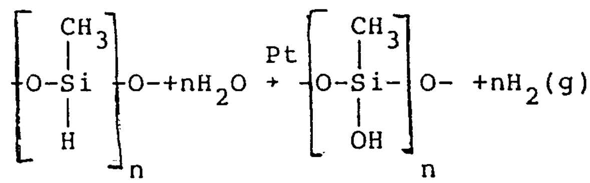


(2)

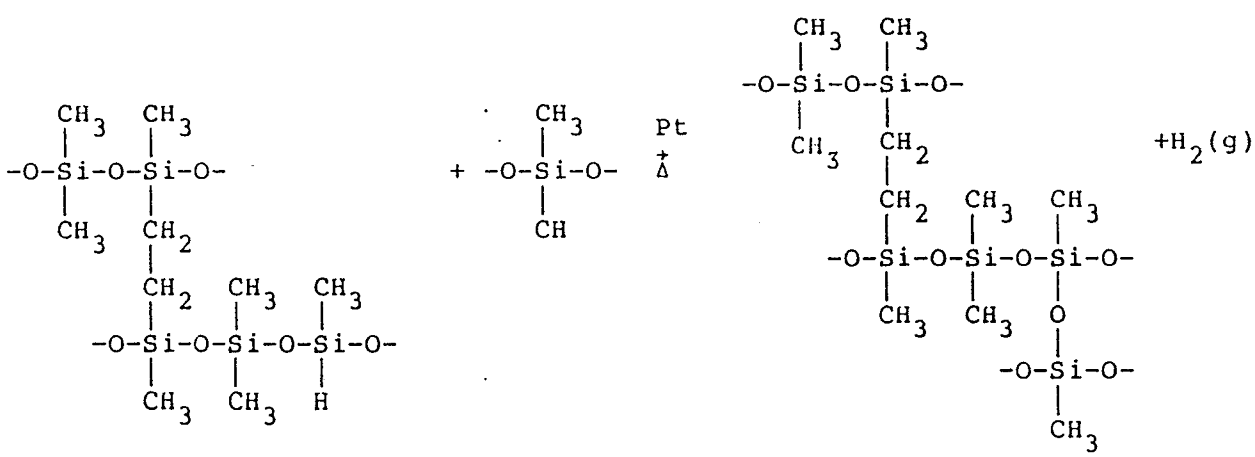


The inventor has discovered that this aging reaction can be substantially eliminated by the deactivation of the catalyst or cured resin. Such deactivation can be accomplished, for example, by subjecting the gel or the article containing the gel to an atmosphere which diffuses into the gel and reacts with the catalyst or cured resin so as to deactivate it. It is believed that platinum (Pt) functions by way of an activate electron-pair acceptor site on the metal atom or ion and, if the availability of that site to accept electrons is removed the catalytic activity can be eliminated. Thus, the catalytic action of platinum can be poisoned or deactivated by reacting the platinum with ammonia. For example, by exposing the gel containing article to an atmosphere having a vapor containing ammonia or by immersion in a solution containing ammonia, and allowing sufficient time for the ammonia to diffuse into the article so as to react with and deactivate the platinum catalyst or

cured resin, one can prevent the catalyst from catalyzing further crosslinking due to the diffusion of water vapor into the article.

In use, the silicone gels may be in the form of an article wherein the gel is encapsulated in a silicone elastomer or other polymeric material so as to be contained in a pouch or pouch-like member. Such is the case with both the prosthesis and the touch sensitive screen previously mentioned.

One method of testing the efficiency of the treatment is to compare the height of the absorption peaks of the Si–H bond at ~2126 $cm^{-1}$ by means of Fourier Transform Infrared Spectroscopy of both treated and untreated samples which have been exposed to water vapor as compared to a freshly prepared silicone gel. If the catalyst has been deactivated such that the exposure to water does not cause further polymerization or crosslinking, the number of Si–H sites should remain constant. Consequently, a sample in which the catalyst has been deactivated and then treated in water vapor would show essentially the same height of the Si–H absorption peak as the freshly prepared sample, while the untreated sample, after exposure to water vapor, would show a greatly reduced height of the Si–H absorption peak due to the reaction of the Si–H bond with the water vapor and ultimately with other Si–H or similar hydroxy groups resulting in further crosslinking.

Another measure of determining the effectiveness of the treatment is to test the force required to achieve a particular amount of deflection of the gel-containing article, e.g., the soft touch screen. Here, if a larger force is required after than before aging, one may conclude that the screen became harder due to further cross-linking. The results of such experiments are shown below.

Touch sensitive screens as shown in Fig. 1 useful in connection with a CRT display as taught in US-Patent 4 346 376 issue to J.B. Mallors or 3 673 327 issued to R.G. Johnson were prepared utilizing a silicone gel prepared by curing a mixture of Dow Corning Silicones which comprises a silicone having a vinyl group in the chain and a silicone having Si–H groups. The screen 10 comprises a thick polyurethane frame 12, a thin polyurethane front cover layer 14 molded to one side of the frame 12, a thin polyurethane back cover layer 16 molded to the opposite side of the frame 12 such that the frame 12 and front and back and front cover layers 14 and 16 form a pouch which is filled with the silicone gel 18.

The effect of aging on screens prepared in this manner was tested by measuring the force required to deflect the screen at a given distance and by observing the change in the ~2126 $cm^{-1}$ peak with respect to a reference peak ~1923 $cm^{-1}$ in the IR spectra.

Ten screens were exposed to ammonia vapors for 24 h. These screens and ten unexposed control screens were placed in an oven at 70 °C to simulate accelerated aging. The hardness of the screens were measured at various times. The average force deflection measured for the unexposed controls and the ammonia exposed screens are given in Table I. It is apparent from the table that the ammonia treatment inhibited hardening.

Table I

| | Before Ammonia Treatment | After Ammonia Treatment | After 66 h at 70 °C | After 122 h at 70 °C |
|---|---|---|---|---|
| Controls | 239.6 | – | 440.9 | 744.9 |
| Ammoniated | 218.4 | 258.1 | 294.9 | 312.0 |

Figs. 2–4, respectively, show a portion of the IR spectra of a control screen prior to any treatment (Fig. 2), a control screen which has been exposed to water (Fig. 3) and a screen which was treated with ammonia vapor for 24 h prior to treatment with water (Fig. 4). As can be seen from the Figures, the relative absorption of the Si–H bond at ~2126 $cm^{-1}$, and that of the reference peak, ~1923 $cm^{-1}$, are the same for the control and the ammonia-treated sample indicating essentially no change in the concentration of Si–H bonds after exposure to water. However, the sample which was not pre-treated with ammonia shows a reversal with respect to the reference peak. This indicates further polymerization resulting in fewer remaining Si–H bonds.

It should be further understood that while this discovery is particularly suitable for maintaining the consistency of a silicone gel, it can also be utilized to prevent further curing of silicone elastomers where there are unreacted Si–H bonds in the elastomer or wherever the combination of water vapor together with the catalyst can cause further polymerization or crosslinking to an extent which is undesirable.

## Claims

1. A method of fabricating an article containing a cured silicon polymer, which method comprises curing a silicone polymer in the presence of a platinum catalyst and limiting the continued curing of the silicon polymer after the desired consistency of the cured material is reached, characterized in that said limiting step comprises exposing the cured polymer to ammonia, which diffuses into the cured polymer and deactivates the platinum catalyst.

2. The method according to claim 1, characterized in that the cured silicone polymer is a silicone gel.

3. The method according to claim 2, characterized in that the ammonia is in the form of a vapor.

4. The method according to claim 2 or 3, characterized in that said silicone gel is contained in a supporting polymeric container forming said article of manufacture, wherein said polymeric container is permeable to ammonia vapors.

5. The method according to claim 4, characterized in that said article of manufacture is a touch sensitive screen.

6. The method according to claim 4, characterized in that said article of manufacture is a prosthesis for implanting into a living body.

**Patentansprüche**

1. Verfahren zur Herstellung eines Gegenstandes, das ein gehärtetes Siliciumpolymer enthält, wobei ein Siliciumpolymer in Anwesenheit eines Platinkatalysators gehärtet wird und das fortgesetzte Aushärten des Siliciumpolymers begrenzt wird, nachdem die gewünschte Konsistenz des gehärteten Materials erreicht ist, dadurch gekennzeichnet, daß die Begrenzung des Aushärtens die Maßnahme enthält, das gehärtete Polymer diffundiert und den Platinkatalysator deaktiviert.

2. Verfahren nach Anspruch 1, dadurch gekennzeichnet, daß das gehärtete Siliciumpolymer ein Siliciumgel ist.

3. Verfahren nach Anspruch 2, dadurch gekennzeichnet, daß Ammoniak dampfförmig ist.

4. Verfahren nach Anspruch 2 oder 3, dadurch gekennzeichnet, daß das Silicongel in einem tragenden Polymeraufnehmer enthalten ist, der den Gegenstand der Herstellung bildet, wobei der Polymeraufnehmer von Ammoniakdämpfen durchdringbar ist.

5. Verfahren nach Anspruch 4, dadurch gekennzeichnet, daß der Gegenstand der Herstellung ein berührungsempfindlicher Bildschirm ist.

6. Verfahren nach Anspruch 4, dadurch gekennzeichnet, daß der Gegenstand der Herstellung eine Prothese für die Implantation in einen lebenden Körper ist.

**Revendications**

1. Procédé de production d'un objet contenant un polymère de silicone durci, qui consiste à durcir un polymère de silicone en la présence d'un catalyseur au platine, et à limiter le durcissement qui se poursuit du polymère de silicone, après que la consistance souhaitée du matériau durci a été atteinte, caractérisé en ce que le stade de limitation consiste à exposer le polymère durci à de l'ammoniac qui diffuse dans le polymère durci et désactive le catalyseur au platine.

2. Procédé suivant la revendication 1, caractérisé en ce que le polymère de silicone durci est un gel de silicone.

3. Procédé suivant la revendication 2, caractérisé en ce que l'ammoniac est sous la forme d'une vapeur.

4. Procédé suivant la revendication 2 ou 3, caractérisé en ce que le gel de silicone est contenu dans un récipient polymère support qui forme l'objet à produire, le récipient polymère étant perméable aux vapeurs d'ammoniac.

5. Procédé suivant la revendication 4, caractérisé en ce que l'objet à produire est un écran sensible au toucher.

6. Procédé suivant la revendication 4, caractérisé en ce que l'objet à produire est une prothèse destinée à être implantée dans un organisme vivant.

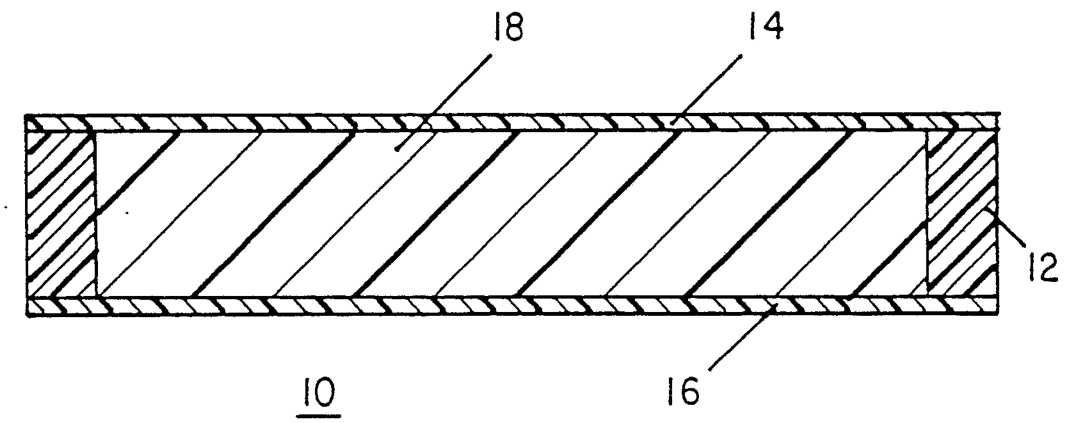


Fig 1

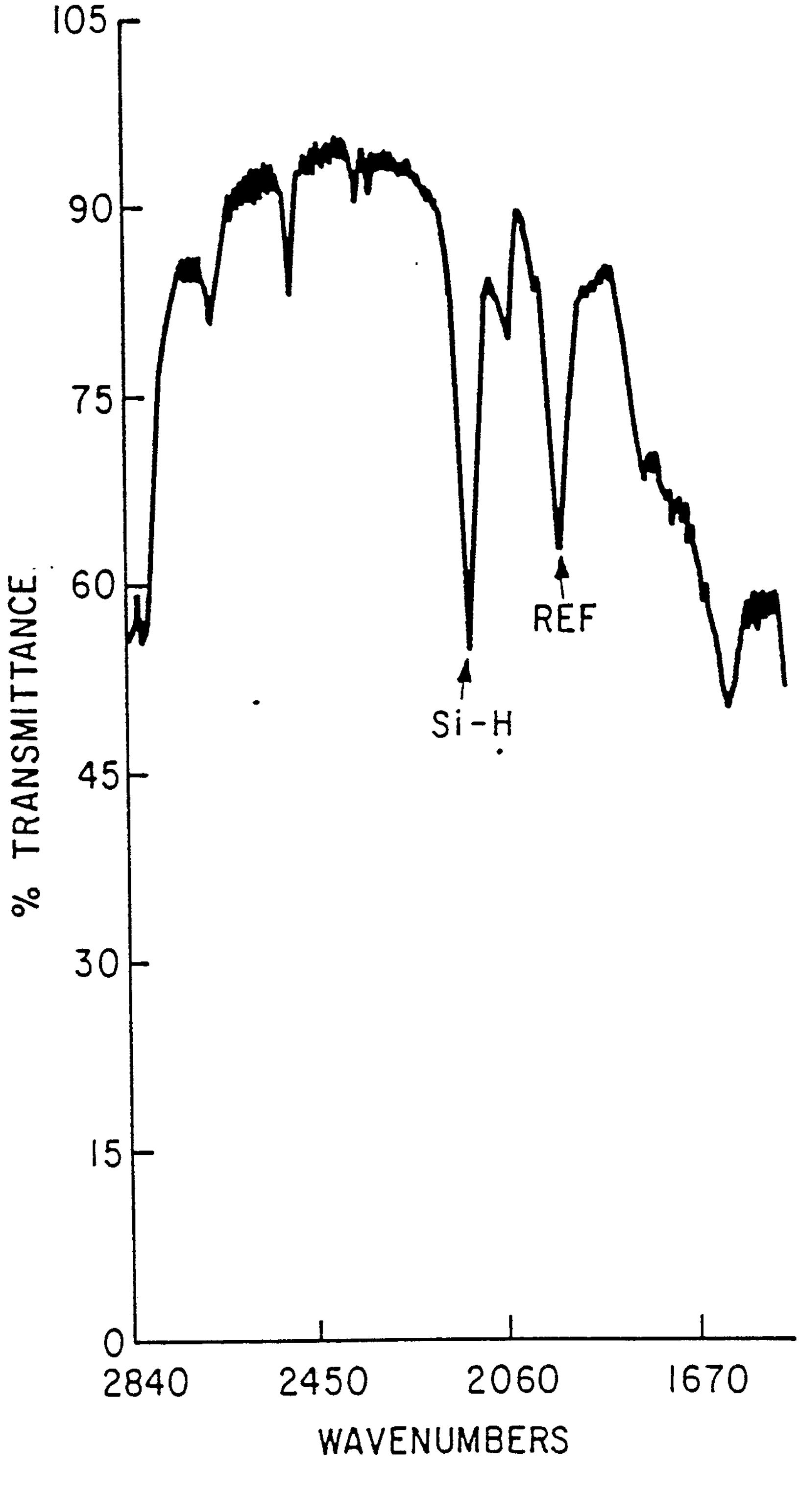
105
90
75
60
45
30
15
0
% TRANSMITTANCE
REF
Si-H
2840
2450
2060
1670
WAVENUMBERS

Fig 2

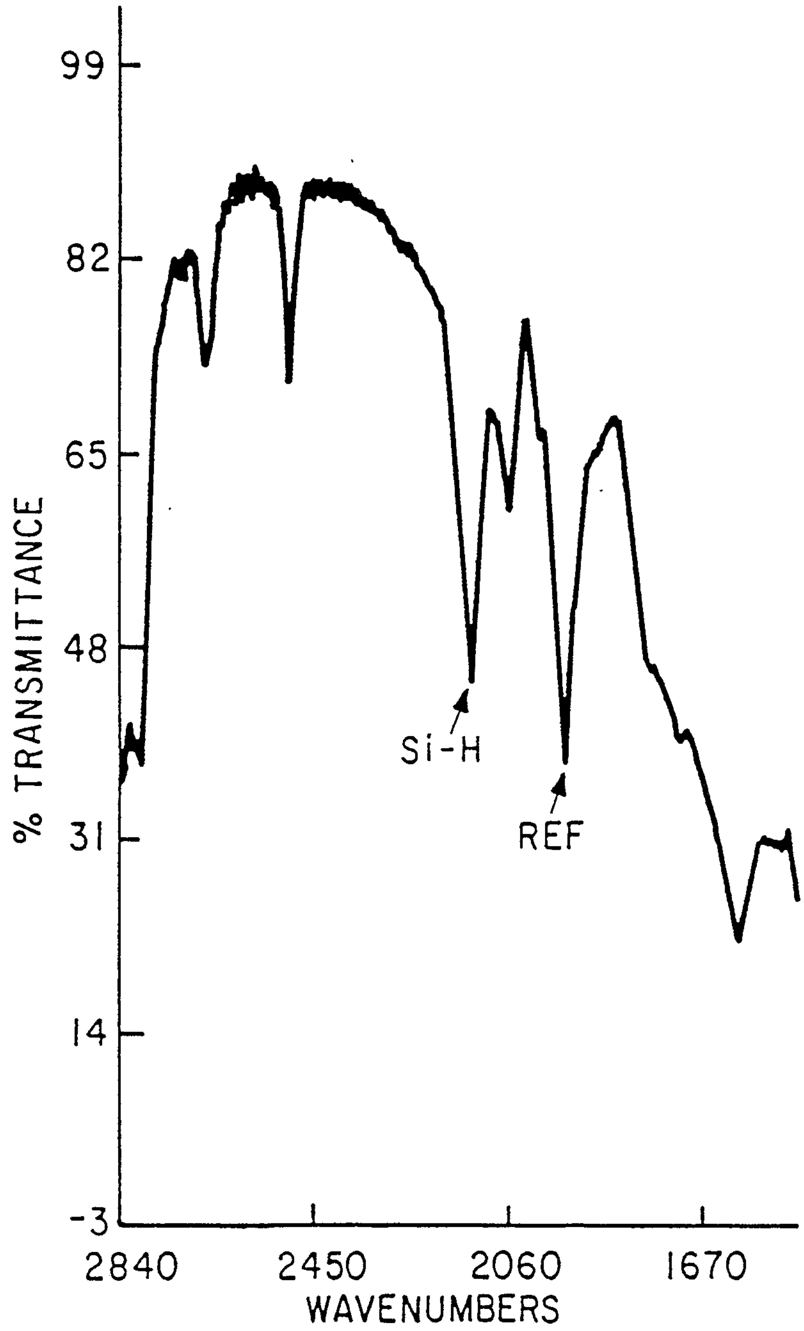
99
82
65
48
31
14
-3
% TRANSMITTANCE
Si-H
REF
2840
2450
2060
1670
WAVENUMBERS

Fig 3

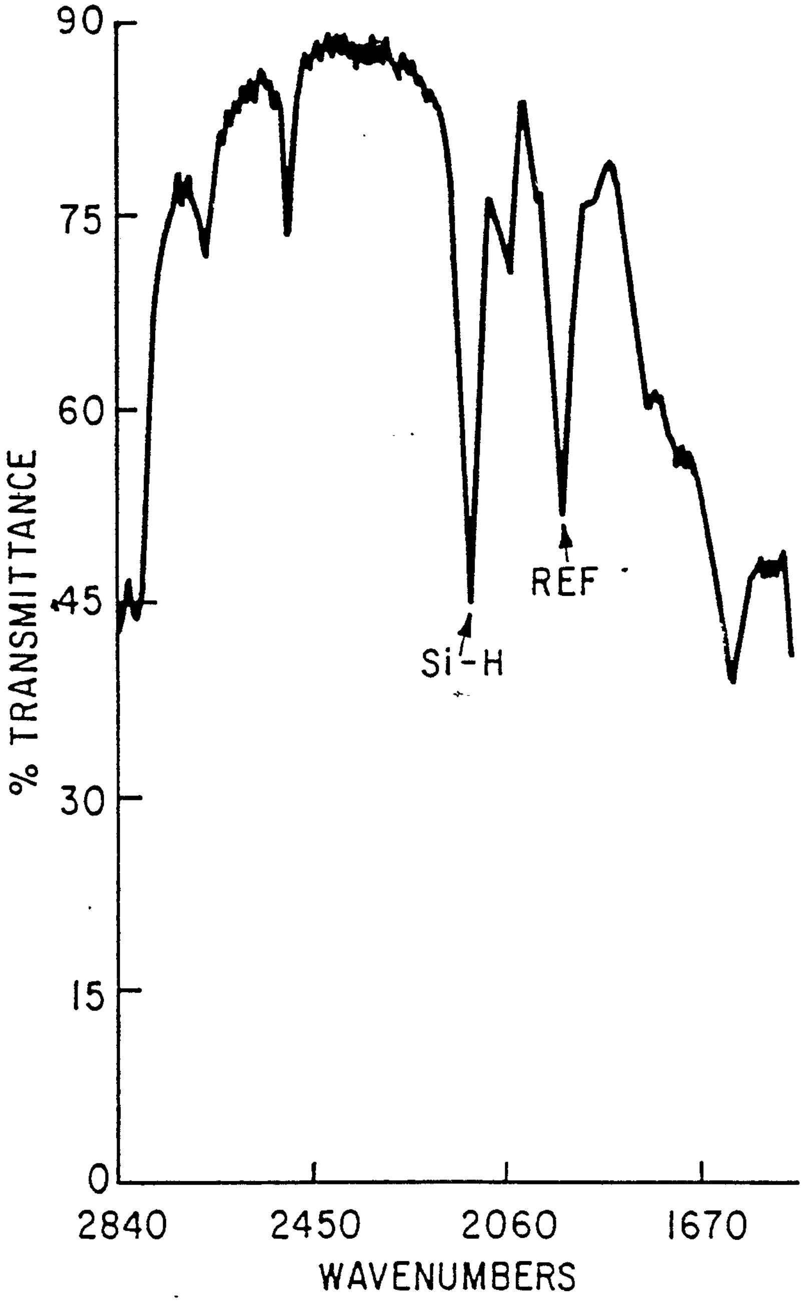
90
75
60
45
30
15
0
% TRANSMITTANCE
2840
2450
2060
1670
WAVENUMBERS
Si-H
REF

Fig 4